# EUROPEAN PATENT APPLICATION

(11) **EP 1 935 493 A2**
(43) Date of publication of application: **25.06.2008**
(21) Application number: 07119467.4
(22) Date of filing: 29.10.2007
(51) Int. Cl.: B01L 3/00, G01N 1/28, A61B 10/00

(54) **Container for samples of organic tissue**

(30) Priority: 15.11.2006 IT PD20060423
(71) Applicant: Kaltek S.r.l., 35127 Padova (IT)
(72) Inventor: Cortelazzo, Lorenzo, 35133, Padova (IT)
(74) Representative: Modiano, Micaela Nadia

(57) **Abstract**

A container for samples of organic tissue, comprising:
- a vessel (11) with an opening (12) which can be sealed by means of a stopper (13);
- one or more cups (14) for containing samples of organic tissue, mutually stacked inside the vessel (11);
- means (15) to allow the distribution of preservative liquid inside the vessel (11) and inside the cups (14).

## Description

The present invention relates to a container for samples of organic tissue.

As is known, in taking samples of organic tissue it is often necessary to take multiple samples in series, such as for example biopsies performed in an operating room, during surgical procedures, or even biopsies performed in an outpatient facility.

Regardless of the sampling techniques used, the number of fragments of samples to be handled for each individual biopsy can be very large.

Such sample fragments must be handled with adequate accuracy and delicacy and must be studied appropriately by viewing with a microscope, preserved, transferred and identified safely.

Drawbacks which can occur during sample handling are for example the loss of fragments during preservation or during transport of said samples to the analysis laboratory.

These drawbacks arise mainly from the fact that the most disparate containers, typically too large for the individual sample, such as bottles, jars, tubes of various shapes and sizes, are used to store the fragments.

Moreover, in other collection situations, all the tissue fragments are collected in a single container.

This inevitably leads to confusion and difficulty in identification as well as difficulty in operation and diagnosis.

Other problems arise when the fragments reach the laboratory.

The analyst must in fact sort the samples and, depending on their macroscopic characteristics, choose the one or the ones to be sectioned for analysis.

The haphazard condition of the samples inside the containers and the dimensions of said samples certainly do not aid the analyst in these operations, which are in any case very important for the satisfactory outcome of the analysis.

Once the samples have been analyzed, they are again taken and transferred to a subsequent treatment for inclusion and generation of slides.

Another problem which occurs in the handling of samples arises from the fact that they are preserved within containers which are immersed in preservative liquids (such as for example formalin).

To take the sample, it is often necessary to overturn the container to pour out the formalin, with the risk of dirtying with said formalin the hands that hold the container.

The aim of the present invention is to solve the problems found in treating samples of organic tissue for diagnostic purposes.

Within this aim, an object of the present invention is to provide a container for samples of organic tissue which allows to store the samples tidily.

Another object of the present invention is to provide a container for samples of organic tissue which allows to view the samples more easily both during storage and during taking.

Another object of the present invention is to provide a container for samples of organic tissue which is simple to use.

Another object of the present invention is to provide a container for samples of organic tissue which allows one to not be dirtied by the sample preservation liquid.

Another object of the present invention is to provide a container for samples of organic tissue which can be manufactured with known technologies.

This aim and these and other objects, which will become better apparent hereinafter, are achieved by a container for samples of organic tissue, characterized in that it comprises:
- a vessel with an opening which can be sealed by means of a stopper;
- one or more cups for containing samples of organic tissue, mutually stacked inside the vessel;
- means to allow the distribution of preservative liquid inside said vessel and inside said one or more containment cups.

Further characteristics and advantages of the invention will become better apparent from the following detailed description of a preferred but not exclusive embodiment thereof, illustrated by way of non-limiting example in the accompanying drawings, wherein:

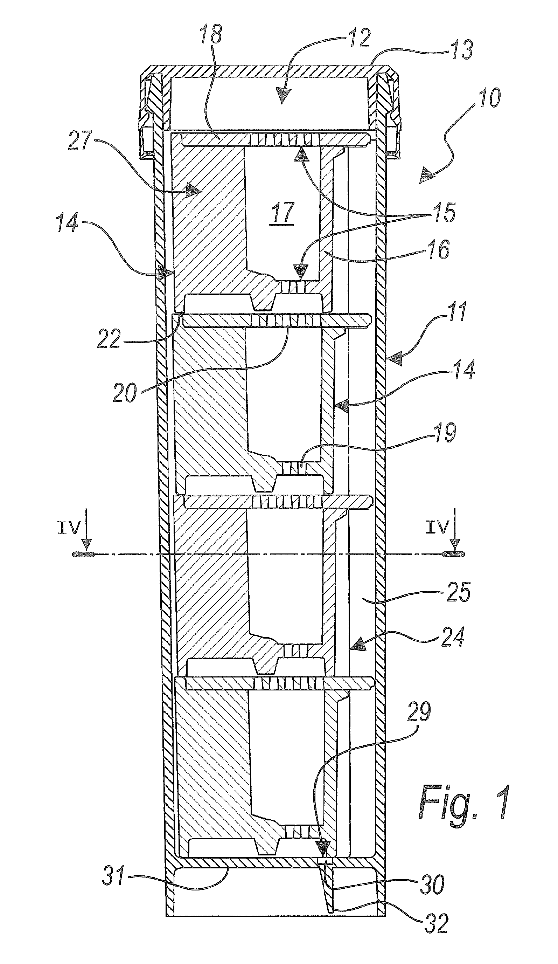

Figure 1 is a longitudinal sectional view of a container according to the invention;

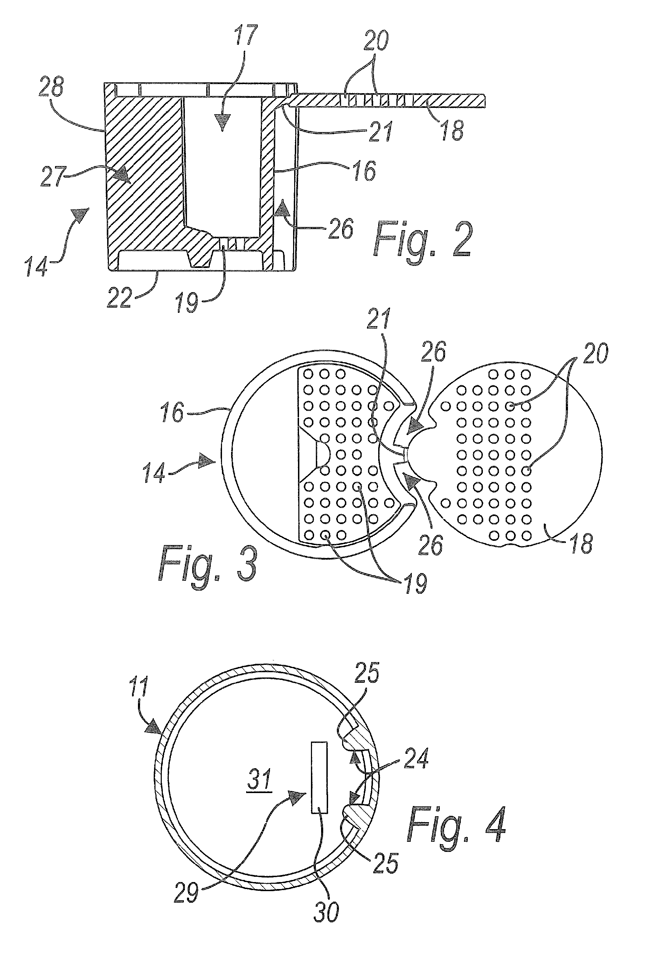

Figure 2 is a longitudinal sectional view of a cup for containing samples of organic tissue which composes the container of Figure 1;
Figure 3 is a plan view of the cup of Figure 2;
Figure 4 is a transverse sectional view of the vessel that composes the container of Figure 1.

It is noted that anything found to be already known during the patenting process is understood not to be claimed and to be the subject of a disclaimer.

With reference to the figures, a container for samples, according to the invention, is generally designated by the reference numeral 10.

The container 10 comprises a vessel 11 with an opening 12 which can be closed by means of a stopper 13; the vessel 11 is elongated upward and, in this embodiment, has a substantially circular cross-section transversely to the longitudinal axis.

The vessel is made of transparent material so as to allow internal viewing.

One or more small mutually stacked containment cups 14 for samples of organic tissue can be arranged inside the vessel 11.

The container 10 further comprises distribution means 15 to allow the distribution of preservative liquid inside the vessel 11 and the cups 14, which are described hereinafter.

Each cup 14 is constituted by a body 16 which forms internally a cavity 17 for accommodating tissue samples.

The cavity 17 of each cup 14 is open at the top and has, in this embodiment, a lid 18 for accessing said cavity.

The distribution means 15 for allowing the distribution of preservative liquid within the vessel 11 and the cups 14 are constituted for example by a plurality of through holes 19 formed in the bottom of the cavity 17 of each cup 14.

Further, in the described version, the distribution means 15 comprise a plurality of additional through holes 20 which are formed in the lid 18.

The through holes 19 and the additional through holes 20 allow the sample preservation liquid to be distributed uniformly within the vessel 11 and the cups 14; once the cups have been inserted in the vessel 11, it is in fact possible to pour the preservative liquid, which strikes the upper cups of the stack, enters them through the additional through holes 20, and exits toward the lower cups by way of the through holes 19 until it fills said vessel.

The lid 18 is preferably monolithic with the body 16 that forms the cavity 17.

In particular, the lid 18 is pivoted to the body 16 by way of a flexible wing 21 (the cup is made for example of plastic material); the lid 18 mates by interlocking with the upper rim of the cavity 17.

Preferably, the supporting base 22 of each cup 14 is substantially flat and parallel to an upper portion 23 of said cup on which an additional cup 14 can be placed in order to form a stack inside the vessel 11.

In particular, each cup 14 protrudes upward substantially at right angles to the supporting base 22.

Conveniently, the container 10 comprises stacking means 24, which are adapted to allow to stack the cups 14 with a same orientation within the vessel 11.

For example, the stacking means 24 are constituted by a rotation-preventing complementary contour of the internal walls of the vessel 11 with respect to the outer side walls of the cups 14.

In particular, the stacking means 24 comprise, on a portion of the inner lateral part of the vessel 11, two parallel ribs 25, which protrude upward and are adapted to mate by insertion with corresponding lateral recesses 26 formed on the cups 14.

Advantageously, each cup 14 comprises means 27 for optically magnifying its contents.

In particular, the optical magnifying means 27 are formed on a side wall 28 of the cavity 17 and are constituted in practice by a portion shaped like a magnifying lens.

Conveniently, the container 10 comprises discharge means 29 for discharging the preservation liquid from the vessel 11, which are separate, located away from the opening 12.

In particular, the means 29 for discharging the preservation liquid comprise a region 30 of the bottom 31 of the vessel 11 which has a weakened structure; a tab 32 is provided on the side of the bottom 31 that lies outside the vessel 11 and is monolithic with the region 30 of the bottom 31 and adapted to be torn to allow to break the region 30 and allow the outflow of the preservative liquid from the vessel 11.

In practice it has been found that the invention thus described achieves its intended aim and objects.

The present invention in fact provides a container for samples of organic tissue which allows to organize tidily the arrangement of the samples.

The samples can in fact be divided appropriately inside the cups, and the cups can be stacked inside the container according to a preset order, which allows the analyst to avoid confusing the samples.

Moreover, the use of cups which have a magnifying lens allows the analyst to identify the samples clearly and not confuse them.

Further, the particular structure of the cups allows optimum filling of the container by the preservative liquid.

The flat base and the right-angled arrangement of said cups allows optimum support thereof on worktables.

The closure lid, which is monolithic with respect to said cups, allows easy handling of the cups even with one hand.

The stacking means allow the cups to maintain a correct orientation within the vessel, thus allowing better viewing of the samples from outside inside the container, as well as easier insertion and extraction of the cups in and from said container.

The presence of means for discharging the preservative liquid on the bottom of the vessel allows to avoid getting dirty while emptying it.

The invention thus conceived is susceptible of numerous modifications and variations, all of which are within the scope of the appended claims; all the details may further be replaced with other technically equivalent elements.

In practice, the materials employed, so long as they are compatible with the specific use, as well as the dimensions, may be any according to requirements and to the state of the art.

The disclosures in Italian Patent Application No. PD2006A000423 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A container for samples of organic tissue, **characterized in that** it comprises:
- a vessel (11) with an opening (12) which can be sealed by means of a stopper (13);
- one or more cups (14) for containing samples of organic tissue, mutually stacked inside said vessel (11);
- means (15) to allow the distribution of preservative liquid inside said vessel (11) and inside said one or more cups (14).

2. The container according to claim 1, **characterized in that** said cup (14) comprises means (27) for optically magnifying its contents.

3. The container according to claim 2, **characterized in that** said cup (14) comprises a body (16) which forms internally a cavity (17) for accommodating samples of tissue, which has, on at least one wall of said cavity (17), said optical magnifying means (27).

4. The container according to claim 3, **characterized in that** with reference to said cup (14), said at least one wall of said cavity (17) with said optical magnifying means (27) is lateral and is constituted by a portion shaped like a magnifying lens.

5. The container according to one or more of the preceding claims, **characterized in that** each of said cups (14) comprises a body (16), which forms internally a cavity (17) for accommodating tissue samples, said cavity (17) of each of said cups (14) having, in an upper region, an openable lid (18) for accessing said cavity (13).

6. The container according to one or more of the preceding claims, **characterized in that** each of said cups (14) comprises a body (16), which forms internally a cavity (17) for accommodating tissue samples, said cavity (17) being open upward, said means (15) for allowing the uniform distribution of preservative liquid inside said vessel (11) and said cups (14) comprising at least one through hole (19) in the bottom of said cavity (17) of each of said cups (14).

7. The container according to claim 5 or 6, **characterized in that** said means (15) for allowing the uniform distribution of preservative liquid within said vessel (11) and said cups (14) further comprise at least one additional through hole (20) formed in said lid (18).

8. The container according to one or more of claims 5, 6 and 7, **characterized in that** said lid (18) is monolithic with said body (16) which forms said cavity (17) and is pivoted to the body (16) of said cup (14) by way of a flexible wing (21), said lid (18) being coupled by interlocking on the upper rim of said cavity (17).

9. The container according to one or more of claims 5, 6 or 7, **characterized in that** the supporting base (22) of said cup (14) is substantially flat and parallel to an upper portion of said cup (14) on which it is possible to rest an additional cup (14) to provide said stack inside said vessel (11).

10. The container according to one or more of the preceding claims, **characterized in that** it comprises means (29) for discharging the preservative liquid from said vessel (11) which are separate from said closable opening (12).

11. The container according to claim 10, **characterized in that** said means (29) for discharging the preservative liquid comprise a region (30) of the bottom (31) of said vessel (11) which has a weakened structure, a tab (32) being arranged on the side of said bottom (31) that lies outside the vessel (11) and being monolithic with respect to said region (30) of the bottom (31) and adapted to be torn to allow the breakage of said region (30) having a weakened structure and allow the outflow of the preservative liquid from the vessel (11).

12. The container according to one or more of the preceding claims, **characterized in that** it comprises stacking means (24) which are adapted to allow the stacking of said cups (14) with a same orientation within said vessel (11).

13. The container according to claim 12, **characterized in that** said stacking means (24) are constituted by a rotation-preventing complementary contour of the inside walls of said vessel (11) with respect to the outer side walls of said one or more cups (14).

14. The container according to claim 13, **characterized in that** said stacking means (24) comprise, on at least one lateral part of said vessel, at least one rib (25) which protrudes upward and is adapted to mate by insertion with a corresponding lateral recess (26) which is formed in said one or more cups (14).

15. The container according to one or more of the preceding claims, **characterized in that** each of said cups (14) protrudes upward substantially at right angles to its supporting base (22).

16. The container according to one or more of the preceding claims, **characterized in that** said vessel (11) is made of transparent material or of material which in any case allows to view its interior.
